# EUROPÄISCHE PATENTSCHRIFT

(11) **EP 3 081 631 B1**
(45) Veröffentlichungstag und Bekanntmachung des Hinweises auf die Patenterteilung: **31.01.2018**
(21) Anmeldenummer: 15001074.2
(22) Anmeldetag: 15.04.2015
(51) Int. Cl.: C12M 1/107, A01C 3/02, B01F 7/02, C12M 1/06, B01F 15/00

(54) **VORRICHTUNG ZUM DURCHMISCHEN DES INHALTS VON SUBSTRATBEHÄLTERN**
DEVICE FOR MIXING THE CONTENT OF SUBSTRATE CONTAINERS
DISPOSITIF DE MÉLANGE DU CONTENU DE RÉCIPIENTS DE SUBSTRATS

(43) Veröffentlichungstag der Anmeldung: 19.10.2016
(73) Patentinhaber: BHKW Johann Hochreiter Biogas Planung Beratung GmbH, 83530 Schnaitsee (DE)
(72) Erfinder: Hochreiter, Johann, 83530 Schnaitsee (DE)

(56) Entgegenhaltungen:
- DE-B- 1 161 860
- DE-U1- 8 312 809
- DE-U1-202006 000 185
- DE-U1-202013 006 429

## Beschreibung

Die vorliegende Erfindung bezieht sich auf eine Vorrichtung zum Durchmischen des Inhalts von Substratbehältern gemäß dem Oberbegriff des Anspruchs 1.

Rührwerke für Biogasanlagen sind bereits bekannt. So ist in der DE 102 60 972 B4 eine Vorrichtung zum Durchmischen von Flüssigmist in einem senkrecht stehenden Vorratsbehälter beschrieben, die sich dadurch auszeichnet, dass eine Welle mit Rührelementen radial in den senkrecht stehenden, zylindrischen Vorratsbehälter hineinragt, mittels einer Antriebsvorrichtung drehbar ist und dass die Rührelemente als schaufelförmige Paddel so ausgebildet sind, dass sie in bestimmten Rotationspositionen einen Durchbruch in einer Abdeckung des Vorratsbehälters durchragen, und dass der Durchbruch mittels einer Haube hermetisch verschließbar ist.

Ferner ist in der DE 10 2008 006 813 A1 eine Rührvorrichtung für Biogasfermenter gezeigt und beschrieben, die zur Wartung oder Reparatur problemlos ohne Veränderung des Füllstands und ohne wesentliche Störung des normalen Betriebsablaufs aus- bzw. eingebaut werden kann. Dazu befindet sich die Rührvorrichtung in einem senkrecht stehenden Schacht, der von oben her in den Fermenterkessel hineinragt und im unteren Teil eine Austrittsöffnung hat. Die Austrittsöffnung liegt dabei etwas unterhalb der Füllstandshöhe, so dass ein gasdichter Abschluss entsteht. Der Schacht mit der Rührvorrichtung kann auch an der Außenseite des Fermenters angebracht sein.

Außerdem ist eine Biogasanlage mit einem Rührwerk aus der DE 20 2006 004 982 U1 bekannt. Dort umfasst das Rührwerk zumindest einen Antrieb und ein an der Wand des Fermentationbehälters befestigtes wandseitiges Rührwellenlager, ein weiteres, sich am Boden abstützendes fermenterseitiges Rührwellenlager und zumindest eine Rührwelle mit Rührblättern. Dabei erstreckt sich die Rührwelle parallel zum Boden des Fermentationsbehälters in Richtung der Hauptachse des Fermentationsbehälters.

Außerdem ist aus der DE 20 2006 020 195 U1 ein Rührwerk für einen Fermenter bekannt, bei dem mehrere vertikal angeordnete Rührwellen Rührpaddel tragen und über einen Antriebsmechanismus zum Antrieb der Rührwellen verfügen. Am Boden des Fermenters befinden sich Zentrierlager zum zentrieren der vertikal angeordneten Rührwellen.

In der DE 20 2004 012 236 U1 ist ein Rührwerk für Biogasfermenter gezeigt und beschrieben, bei dem ein schräg ins innere des Fermentationsbehälters ragendes Rührwerk zwei Rührschaufeln auf seiner Rührwelle trägt. Die Rührschaufeln sind Propellerartig ausgebildet und in gleicher Ausrichtung auf der Rührwelle positioniert. Ihre Rotationskreise überschneiden sich in Bezug auf das Flüssigkeitsniveau im Fermentationsbehälter nicht. Kern des dort beschriebenen Gegenstands ist die speziell ausgestaltete Fermenterseitige Lagerkonsole.

In der DE 83 128 09 U1 ist ein Rührwerk für einen Fermenter gezeigt und beschrieben, bei dem an einer schräg in den Fermenter eingebauten Rührwelle zwei Rührer in übereinander liegenden Ebenen vorgesehen sind, von denen der obere Rührer an einem Schwimmer angeordnet ist. Die Rührer haben propellerartige Rührflügel, die eine Strömung entlang der Längsachse der Rührwelle erzeugen. Die Anordnung der Rührer ist so gewählt, dass die Rührflügel die Oberfläche der zu durchmischenden Gülle nicht durchbrechen. Die Rührflügel sind bei dieser Vorrichtung propellerartig ausgebildet und weisen ein tragflächenartiges Profil auf. Die so erzeugbare Strömung ist als im Wesentlichen lokaler Mischvorgang, also als Verwirbelung anzusehen.

In der DE 20 2013 006429 U1 ist eine Vorrichtung zum Durchmischen des Inhalts von Substratbehältern gezeigt und beschrieben. Das dort gezeigte Rührwerk ist schräg in dem Substratbehälter montiert und weist wenigstens zwei Rührschaufeln auf. Diese Rührschaufeln erstrecken sich beidseits diametral der drehbaren Welle und befinden sich wechselweise in dem Inhalt des Substratbehälters oder ragen aus ihm heraus.

Der vorliegenden Erfindung liegt die Aufgabe zu Grunde, eine Vorrichtung zum Durchmischen des Inhalts von Substratbehältern, insbesondere bei Biogasanlagen zu schaffen, die über eine hohe Leistungsfähigkeit verfügt, sehr gut an die wechselnden Bedingungen beim Betrieb der Biogasanlage anpassbar ist und bei der Wartung geringe Störungen des Betriebs verursacht. Dabei soll nicht nur eine örtliche Verwirbelung des Inhalts erfolgen, sondern der gesamte Inhalt des Substratbehälters soll in Bewegung gebracht werden.

Diese Ausgabe wird von einer Vorrichtung mit den Merkmalen des Anspruchs 1 gelöst. Vorteilhafte Ausgestaltungen der Vorrichtung sind den abhängigen Ansprüchen zu entnehmen.

Die Vorteile der erfindungsgemäßen Vorrichtung für Biogasanlagen liegen in ihrer großen Leistungsfähigkeit, ihrer Stabilität, ihrer Wartungsfreundlichkeit und ihrer Fähigkeit, den gesamten Inhalt des Substratbehälters in Bewegung zu versetzen.

Vorteilhaft ist eine Vorrichtung zum Durchmischen des Inhalts von Substratbehältern mittels eines Rührwerks vor allem dann, wenn der Substratbehälter zumindest eine umlaufende Wand sowie einen Boden umfasst, und wenn sich die umlaufende Wand an einer vertikalen Hauptachse orientiert, das Rührwerk eine Welle umfasst, die unter einem von der Vertikalen und Horizontalen abweichenden Einbauwinkel (α) in den Substratbehälter hineinragt, wenn ferner das Rührwerk mit einem Auftriebskörper verbunden ist und abhängig vom Flüssigkeitsniveau im Substratbehälter in Längserstreckung der Welle auf dieser verschiebbar, aber verdrehfest angeordnet ist, und wenn das Rührwerk wenigstens zwei Rührschaufeln umfasst, die sich diametral beidseits der drehbaren Welle erstrecken und sich während der Drehung wechselweise in dem Inhalt des Substratbehälters befinden bzw. aus ihm herausragen. Die Rührschaufeln sind oberhalb des Auftriebskörpers angeordnet Bei der erfindungsgemäßen Vorrichtung ist es außerdem vorteilhaft, wenn die Welle mehrteilig ausgebildet ist und eine prismatische Außenhülle aufweist, und wenn der Auftriebskörper über eine Führung verfügt, welche die prismatische Außenhülle der Welle aufnimmt.

Günstig ist eine Vorrichtung gestaltet, wenn die Welle des Rührwerks am Boden des Substratbehälters abgestützt ist.

Eine Vorrichtung ist dann vorteilhaft aufgebaut, wenn die Abstützung der Welle am Boden des Substratbehälters mit Hilfe eines Lagerbocks erfolgt.

Günstig gestaltet ist eine Vorrichtung auch dann, wenn die Welle des Rührwerks die Wand des Substratbehälters durchdringt.

Außerdem kann eine Vorrichtung auch dann vorteilhaft sein, wenn die Welle des Rührwerks die Abdeckung des Substratbehälters durchdringt

Eine Vorrichtung ist vor allem dann günstig ausgestaltet, wenn der Substratbehälter als Fermentationsbehälter, Nachgärbehälter, Endlager oder dergleichen ausgebildet ist.

Vorteilhaft ist es bei einer Vorrichtung, wenn der Substratbehälter eine gasdichte Abdeckung aufweist.

Besonders vorteilhaft ist es bei einer Vorrichtung wenn die umlaufende Wand des Substratbehälters in der Draufsicht Kreisringförmig ist.

Außerdem ist eine Vorrichtung von Vorteil, wenn die Rührschaufeln bis in ihre Endbereiche ein sich radial zur Welle erstreckendes Rechteckprofil aufweisen, die Endbereiche gegenüber der Erstreckungsrichtung des Rechteckprofils jedoch Abwinklungen aufweisen.

Mit Hilfe eines Ausführungsbeispiels soll die Erfindung anhand der Zeichnungen noch näher erläutert werden.

Es zeigt
Figur 1 eine Schräg- Teilansicht eines offenen Substratbehälters einer Biogasanlage mit erfindungsgemäßem Rührwerk;
Figur 2 ein Substratbehälter mit Rührwerk in Seitenansicht;
Figur 3 eine Variante des Rührwerks gemäß der Erfindung;
Figur 4 eine Einzelheit eines Auftriebskörpers und
Figur 5 eine Rührwelle als Einzelheit.

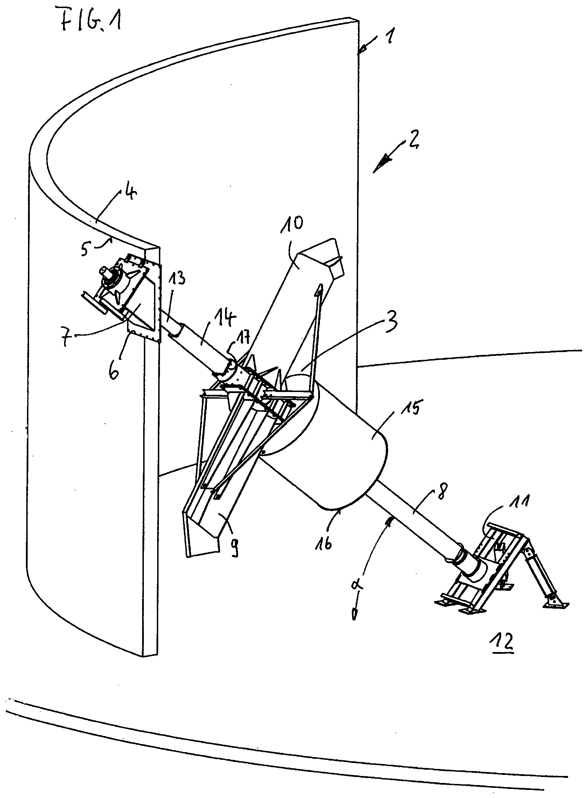

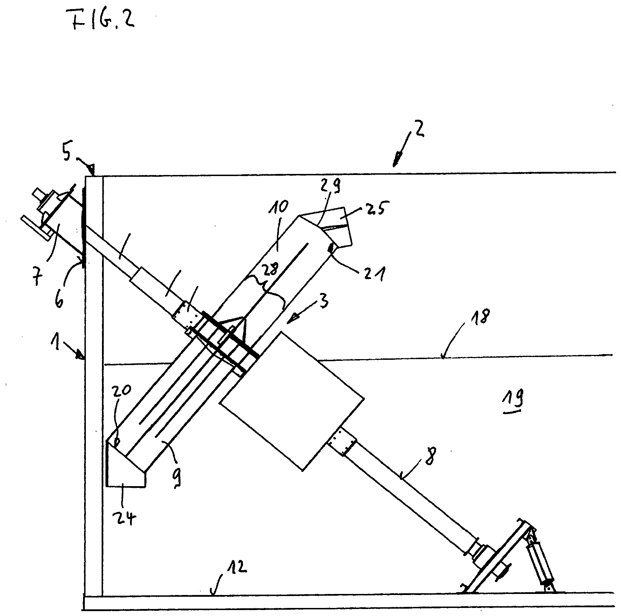

In Figur 1 ist ein offener Substratbehälter gezeigt, der in der folgenden Beschreibung als Fermentationsbehälter 1 einer Biogasanlage 2 bezeichnet wird. Der nicht dargestellte Inhalt soll von einem Rührwerk 3 durchgemischt werden, welches bei diesem Ausführungsbeispiel in einem zylindrischen Fermentationsbehälters 1 angeordnet sind. Eine Wand 4 des Fermentationsbehälters 1 ist vorzugsweise thermisch isoliert und vorzugsweise aus Beton gefertigt. Der obere Begrenzungsrand 5 des Fermentationsbehälters 1 weist eine Verstärkungsplatte 6 auf, mittels der ein Antriebsmotor 7 für eine Rührwelle 8 - gasdicht abgeschirmt - an dem Fermentationsbehälter 1 gelagert ist. Die Rührwelle 8 ragt unter einem von der Vertikalen und Horizontalen abweichenden Einbauwinkel α in den Fermentationsbehälter 1 hinein und trägt zwei Rührschaufeln 9 und 10, welche diametral an der Rührwelle 8 montiert sind. An ihrem dem Antriebsmotor 7 abgewandten Ende ist die Rührwelle 8 in einem Lagerbock 11 abgestützt, der sich am Boden 12 des Fermentationsbehälters 1 befindet. Die Rührwelle 8 ist mehrteilig ausgeführt und besteht aus einer vom Antriebsmotor 7 angetriebenen Welle 13 und einer prismatischen Außenhülle 14, welche die Welle 13 umgibt. Auf der prismatischen Außenhülle 14 ist ein Auftriebskörper 15 längsverschieblich gelagert. Der Auftriebskörper 15 besteht aus einem hohlen, zylindrischen Schwimmkörper 16, dessen zentrale Längsachse durch eine Führung 17 für die prismatische Außenhülle 14 der Rührwelle 8 realisiert ist. Der Auftrieb des Schwimmkörpers 16 ist so bemessen, dass er dem Flüssigkeitsniveau 18 des Substrats 19 (s. Figur 2) folgt. Da die Rührschaufeln 9 und 10 oberhalb des Schwimmkörpers 16 angeordnet sind, ergibt sich aus dieser Bauweise, dass sich immer eine Rührschaufel 9 oder 10 innerhalb und die andere außerhalb des Substrats 19 befindet. Die Folge dieser Gegebenheit ist ein rhythmisches Eintauchen der Rührschaufeln 9 und 10 in das Substrat 19, was zu einer Wogenbildung des Substrats 19 im Fermentationsbehälter 1 führt. Diese Woge rotiert letztlich innerhalb des Fermentationsbehälters 1 und so bewegt sich das gesamte Substrat 19 in Form einer Woge durch den Fermentationsbehälter 1.

In Figur 2 ist das Rührwerk 3 in Seitenansicht gezeigt. Um Wiederholungen zu vermeiden sind gleiche oder gleich wirkende Bauteile mit den gleichen Bezugszeichen versehen, wie in Figur 1, auf die ausdrücklich Bezug genommen wird. Mit Hilfe des Antriebsmotors 7 wird die Rührwelle 8 in Rotation versetzt und die Rührschaufeln 9 und 10 versetzen das im Fermentationsbehälter 1 befindliche Substrat 19 (dessen Flüssigkeitsniveau hier als Linie 18 dargestellt ist) in Bewegung. Je nach Füllstand des Fermentationsbehälters 1 tauchen die Rührschaufeln 9 und 10 - wie vorbeschrieben - dabei mehr oder weniger in das Substrat 19 ein. Gegenüber dem aus dem Stand der Technik bekannten Propeller-Rührwerken wird das Substrat 19 nicht nur örtlich am Propeller verwirbelt, sondern die Gesamtheit des Substrats 19 wird durch die Rührschaufeln 9 und 10 innerhalb des Fermentationsbehälters 1 in Rotation versetzt. Es ist ersichtlich, dass die Rührschaufeln 9 und 10 an ihren Endbereichen 20, 21, 22 und 23 Abwinklungen 24, 25, 26 und 27 aufweisen. Die Rührschaufeln 9 und 10 sind bevorzugt aus Stahlblech hergestellt und prismatisch abgekantet, so dass sich in Rührrichtung - die der Drehrichtung der Rührwelle 8 entspricht - jeweils ein prismatisches Profil 28 mit dem Querschnitt eines Schiffsbugs 29 ergibt, wodurch beim Rühren im Substrat 19 die Kräfte gering gehalten werden. Die Abwinklungen 24, 25, 26 und 27 optimieren die Form der Rührschaufeln 9 und 10 dahingehend, dass die wirksamen Flächen der Rührschaufeln 9 und 10 möglichst nahe an die Randbereiche des Fermentationsbehälters 1 heranreichen, wobei sie in diesen Bereichen ihre Rührwirkung beibehalten, weil der Schaufeleffekt stärker ist, als bei einem Propellerartig rund auslaufenden Ende der Rührschaufeln gemäß dem Stand der Technik+. Dies gilt sowohl für die Bereiche der Wand 4 als auch für den Boden 12 des Fermentationsbehälters 1.

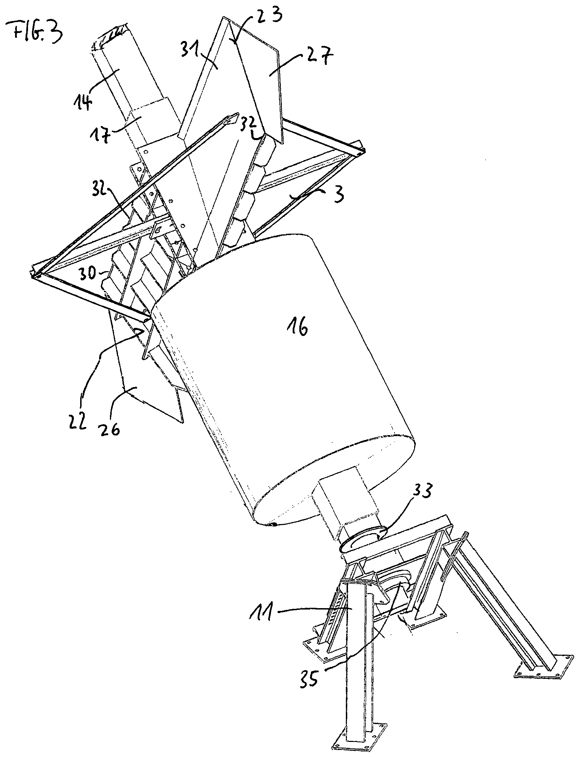

In Figur 3 ist ein Rührwerk 3 in einer Variante dargestellt, bei der die Rührschaufeln 30 und 31 den Querschnitt einer schmalen Klinge 32 aufweisen. Die Positionierung oberhalb des Schwimmkörpers 16 und die Ausrichtung der Klingen 32 zu dessen Längsachse ist so gewählt, dass sich ein möglichst widerstandarmes Eintauchen in das Substrat 19 ergibt. Gegenüber der Darstellungen in den Figuren 1 und 2 sind also die Rührschaufeln 30 und 31 anders ausgestaltet obwohl die gleiche Wogenbewegung des Substrats 19 wie bei der Vorrichtung gemäß der Figur 1 als auch der Figur 2 erzielt wird.

Durch die erfindungsgemäße Ausbildung der Rührwerke und deren Wirksamkeit im Oberflächenbereich der Güllefüllung ist auch bei sich ändernder Füllhöhe - also in der Region des Flüssigkeitsniveaus - gewährleistet, dass die Bildung von Schwimmdecken verhindert wird.

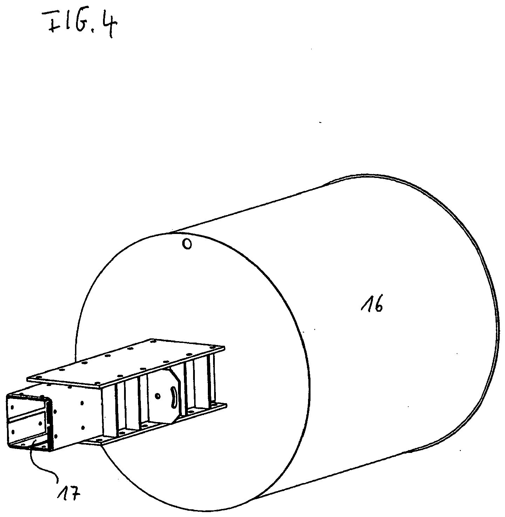

In Figur 4 ist schematisch der Auftriebskörper 15 dargestellt, der als zylindrischer Hohlkörper den Schwimmkörper 16 bildet. In seiner Längsachse befindet sich eine Führung 17, in welcher die prismatische - hier quadratische - Außenhülle 14 der Rührwelle 8 längsverschieblich gelagert ist. Die Längsverschieblichkeit ermöglicht dem Schwimmkörper 16 mit seinem Rührwerk 3 dem Flüssigkeitsniveau 18 des Substrats 19 zu folgen. Die prismatische Ausführung der Außenhülle 14 und deren Verdrehfestigkeit in der Führung 17 bewirkt die drehsteife Mitnahme des Rührwerks 3 auf der Rührwelle 8. Es versteht sich, dass der Schwimmkörper auch als anders ausgestalteter Auftriebskörper realisiert sein kann.

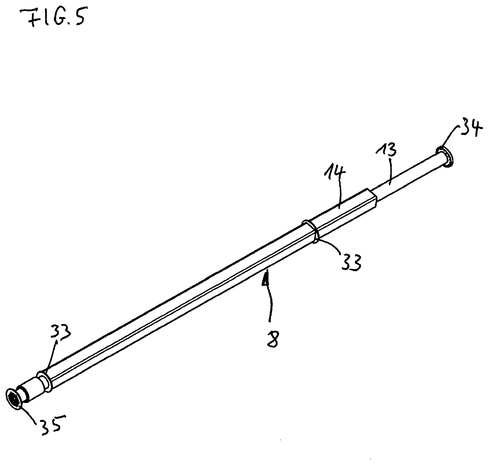

In Figur 5 ist schließlich die Rührwelle 8 mit ihren wesentlichen Details dargestellt. Als Kern ist eine Welle 13 vorhanden, auf welcher eine prismatische - hier quadratische - Außenhülle 14 angeordnet ist. An der Außenhülle befindet sich wenigstens ein Anschlag 33, der den Schwimmkörper 16 in seiner axialen Bewegung begrenzt. Flansche 34 und 35 dienen zur Befestigung der Rührwelle 8 am Antriebsmotor 7 und am Lagerbock 11.

### Bezugszeichenliste

- 1: Fermentationsbehälter (Substratbehälter).
- 2: Biogasanlage
- 3: Rührwerk
- 4: Wand
- 5: Oberer Begrenzungsrand
- 6: Verstärkungsplatte
- 7: Antriebsmotor
- 8: Rührwelle
- 9: Rührschaufel
- 10: Rührschaufel
- 11: Lagerbock
- 12: Boden
- 13: Welle
- 14: Außenhülle
- 15: Auftriebskörper
- 16: Schwimmkörper
- 17: Führung
- 18: Flüssigkeitsniveau
- 19: Substrat
- 20: Endbereich
- 21: Endbereich
- 22: Endbereich
- 23: Endbereich
- 24: Abwinklung
- 25: Abwinklung
- 26: Abwinklung
- 27: Abwinklung
- 28: Profil
- 29: Schiffsbug
- 30: Rührschaufel
- 31: Rührschaufel
- 32: Klinge
- 33: Anschlag
- 34: Flansch
- 35: Flansch

## Patentansprüche

1. Vorrichtung zum Durchmischen des Inhalts von Substratbehältern (1) mittels eines Rührwerks (3), bei der der Substratbehälter (1) zumindest eine umlaufende Wand (4) und ei nen Boden (12) umfasst, wobei sich die umlaufende Wand (4) an einer vertikalen Hauptachse orientiert, und das Rührwerk (3) eine Rührwelle (8) umfasst, die unter einem von der Vertikalen und Horizontalen abweichenden Einbauwinkel (a) in den Substratbehälter (1) hineinragt, wobei das Rührwerk (3) wenigstens zwei Rührschaufel (9, 10) umfasst, die sich diametral beidseits der drehbaren Rührwelle (8) erstrecken und sich wechselweise in dem Inhalt des Substratbehälters (1) befinden bzw. aus ihm herausragen, **dadurch gekennzeichnet, dass** das Rührwerk (3) einen Auftriebskörper (15) aufweist, der abhängig vom Flüssigkeitsniveau (18) im Substratbehälter (1) in Längserstreckung der Rührwelle (8) auf dieser verschiebbar, aber verdrehfest angeordnet ist, und das wenigstens zwei Rührschaufeln (9, 10; 30, 31) oberhalb des Auftriebskörpers (15) angeordnet und mit diesem verbunden sind.

2. Vorrichtung nach Anspruch 1, **dadurch gekennzeichnet, dass** die Rührschaufeln (9, 10) den Querschnitt eines Schiffsbugs (29) aufweisen.

3. Vorrichtung nach Anspruch 1, **dadurch gekennzeichnet, dass** die Rührschaufeln (30, 31) den Querschnitt einer Klinge (32) haben.

4. Vorrichtung nach Anspruch 1, **dadurch gekennzeichnet, dass** die Rührwelle (8) mehrteilig aus einer Welle (13) mit einer prismatische Außenhülle (14) gebildet ist, und dass der Auftriebskörper (15) über eine Führung (17) verfügt, welche die prismatische Außenhülle (14) der Welle (13) aufnimmt.

5. Vorrichtung nach Anspruch 1, **dadurch gekennzeichnet, dass** die Rührwelle (8) des Rührwerks (3) am Boden (12) des Substratbehälters (1) abgestützt ist.

6. Vorrichtung nach Anspruch 5, **dadurch gekennzeichnet, dass** die Abstützung der Rührwelle (8) am Boden (12) des Substratbehälters (1) mit Hilfe eines Lagerbocks (11) erfolgt.

7. Vorrichtung nach Anspruch 1, **dadurch gekennzeichnet, dass** die Rührwelle (8) des Rührwerks (3) die Wand (4) des Substratbehälters (1) durchdringt.

8. Vorrichtung nach Anspruch 1, **dadurch gekennzeichnet, dass** die Rührwelle (8) des Rührwerks (3) eine nicht dargestellte Abdeckung des Substratbehälters (1) durchdringt

9. Vorrichtung nach Anspruch 1, **dadurch gekennzeichnet, dass** der Substratbehälter (1) als Fermentationsbehälter, Nachgärbehälter, Endlager oder dergleichen ausgebildet ist.

10. Vorrichtung nach Anspruch 1, **dadurch gekennzeichnet, dass** der Substratbehälter (1) eine gasdichte Abdeckung aufweist.

11. Vorrichtung nach Anspruch 1, **dadurch gekennzeichnet, dass** die umlaufende Wand (4) des Substratbehälters (1) in der Draufsicht Kreisringförmig ist.

## Claims

1. Device for mixing the contents of substrate containers (1) using a stirrer (3), in which the substrate container (1) has at least one circulating wall (4) and a base (12), the circulating wall (4) being aligned on a vertical main axis, and the stirrer (3) contains a stirrer shaft (8) which projects into the substrate container (1) at an angle (α) that deviates from the vertical and horizontal, the stirrer (3) having at least two stirring paddles (9, 10) which stretch diametrically on both sides of the rotating stirrer shaft (8) and, alternately, are positioned in the content of the substrate container (1) or stick out from it, **characterised by** the fact that the stirrer (3) has a float (15) which, depending on the liquid level (18) in the substrate container (1), extends along the length of the stirrer shaft (8) so that it can be slid along it but not rotated, and at least two stirring paddles (9, 10; 30, 31) are arranged above the float (15) and connected to it.

2. Device as per claim 1, **characterised by** the fact that the stirring paddles (9, 10) exhibit the cross-section of a ship's bow (29).

3. Device as per claim 1, **characterised by** the fact that the stirring paddles (30, 31) exhibit the cross-section of a blade (32).

4. Device as per claim 1, **characterised by** the fact that the stirrer shaft (8) is a composite of a shaft (13) with a prismatic outer casing (14), and that the float (15) has a guide (17) that receives the prismatic outer casing (14) of the shaft (13).

5. Device as per claim 1, **characterised by** the fact that the stirrer shaft (8) of the stirrer (3) is supported at the base (12) of the substrate container (1).

6. Device as per claim 5, **characterised by** the fact that the support of the stirrer shaft (8) on the base (12) of the substrate container (1) is done with a bearing block (11).

7. Device as per claim 1, **characterised by** the fact that the stirrer shaft (8) of the stirrer (3) penetrates the wall (4) of the substrate container (1).

8. Device as per claim 1, **characterised by** the fact that the stirrer shaft (8) of the stirrer (3) penetrates a cover (not shown) of the substrate container (1).

9. Device as per claim 1, **characterised by** the fact that the substrate container (1) is formed as a fermentation container, post-fermentation container or final repository or the like.

10. Device as per claim 1, **characterised by** the fact that the substrate container (1) has an airtight cover.

11. Device as per claim 1, **characterised by** the fact that the circulating wall (4) of the substrate container (1) has a circular ring shape in the top view.

## Revendications

1. Dispositif pour mélanger les contenus des récipients du substrat (1) à l'aide d'un agitateur (3), dans lequel le récipient du substrat (1) a au moins une paroi de circulation (4) et une base (12), la paroi de circulation (4) étant alignée sur un axe principal vertical, et l'agitateur (3) contient un arbre d'agitation (8) qui fait saillie dans le récipient du substrat (1) selon un angle (a) qui s'écarte de la verticale et de l'horizontale, l'agitateur (3) ayant au moins deux pales d'agitation (9, 10) s'étendant diamétralement de part et d'autre de l'arbre d'agitation rotatif (8) et positionnées alternativement dans le contenu du récipient du substrat (1) ou ressortant de celui-ci, **caractérisé par le fait que** l'agitateur (3) comporte un flotteur (15) qui, en fonction du niveau de liquide (18) dans le récipient de substrat (1), s'étend sur la longueur de l'arbre d'agitation (8) de manière à pouvoir glisser le long de celui-ci et au moins deux palettes d'agitation (9, 10; 30, 31) sont agencées au-dessus du flotteur (15) et reliées à celui-ci.

2. Dispositif selon la revendication 1, **caractérisé par le fait que** les palettes d'agitation (9, 10) présentent la section transversale de l'étrave d'un navire (29).

3. Dispositif selon la revendication 1, **caractérisé par le fait que** les palettes d'agitation (30, 31) présentent la section transversale d'une lame (32).

4. Dispositif selon la revendication 1, **caractérisé par le fait que** l'arbre d'agitation (8) est un composite d'un arbre (13) avec une enveloppe extérieure prismatique (14), et que le flotteur (15) a un guide (17) qui reçoit l'enveloppe extérieure prismatique (14) de l'arbre (13).

5. Dispositif selon la revendication 1, **caractérisé par le fait que** l'arbre d'agitation (8) de l'agitateur (3) est supporté à la base (12) du récipient du substrat (1).

6. Dispositif selon la revendication 5, **caractérisé par le fait que** le support de l'arbre d'agitation (8) sur la base (12) du conteneur du substrat (1) est réalisé avec un palier (11)

7. Dispositif selon la revendication 1, **caractérisé par le fait que** l'arbre d'agitation (8) de l'agitateur (3) pénètre dans la paroi (4) du récipient du substrat (1).

8. Dispositif selon la revendication 1, **caractérisé par le fait que** l'arbre d'agitation (8) de l'agitateur (3) pénètre dans un couvercle (non représenté) du récipient du substrat (1) .

9. Dispositif selon la revendication 1, **caractérisé par le fait que** le récipient du substrat (1) est formé comme un récipient de fermentation, un récipient de post-fermentation ou un dépôt final ou analogue.

10. Dispositif selon la revendication 1, **caractérisé par le fait que** le récipient du substrat (1) comporte un couvercle étanche à l'air.

11. Dispositif selon la revendication 1, **caractérisé par le fait que** la paroi de circulation (4) du récipient du substrat (1) a une forme d'anneau circulaire dans la vue de dessus.
